# EUROPEAN PATENT SPECIFICATION

(11) **EP 2 906 159 B1**
(45) Date of publication and mention of the grant of the patent: **01.03.2017**
(21) Application number: 12784492.6
(22) Date of filing: 12.10.2012
(51) Int. Cl.: A61F 9/02, F41H 1/00, G01S 7/48

(54) **PERSONAL LASER PROTECTION DEVICE**
PERSÖNLICHE LASERSCHUTZVORRICHTUNG
DISPOSITIF DE PROTECTION ANTI-LASER PERSONNEL

(43) Date of publication of application: 19.08.2015
(73) Proprietor: Fraunhofer-Gesellschaft zur Förderung der angewandten Forschung e.V., 80686 München (DE)
(72) Inventor: RITT, Gunnar, 76131 Karlsruhe (DE); EBERLE, Bernd, 78462 Konstanz (DE); ANSTETT, Gregor, 76676 Graben-Neudorf (DE); EBERT, Reinhard, 72411 Bodelshausen (DE); BEYERER, Jürgen, 69234 Diehlheim (DE)
(74) Representative: Friese Goeden Patentanwälte PartGmbB
(86) International application number: PCT/EP2012/070258
(87) International publication number: WO 2014/056543

(56) References cited:
- EP-A2- 1 459 715
- GB-A- 2 361 056
- US-A- 4 462 661
- US-A- 4 494 835
- US-A- 5 828 437
- US-H- H 686

## Description

### FIELD OF INVENTION

The invention relates to a personal laser protection device comprising fixing means being adapted to be worn on a user's head and being adapted to carry a protective filter. Devices of this type may be used by working staff to protect their eyes from powerful laser radiation which may harm a user's eye.

### BACKGROUND OF INVENTION

From DE 202005007153 eye glasses having a radiation protection filter are known. The protection filter is arranged between a laser source and the eye of the user wearing said eye glasses. Incident laser radiation is absorbed by the protection filter while radiation comprising another wavelength may be transmitted thereby not constraining the user's view.

This known device has the disadvantage that the protection filter absorbs a small range of wavelengths only. This means, that the wavelength and therefore the type of the laser the user has to be protected from must be known in advance. This may be easy in a laboratory working with laser radiation. However, pilots, policemen or other target persons are more and more attacked by small portable yet powerful laser sources which are easily available. These laser sources may have different wavelengths so that a potential target person is not aware of the wavelengths of incident laser radiation or the direction from which the attack is made.

US 4462661 refers to a laser ray protection device comprised of a laser detector, voltage controlled lens or lenses, and related interconnecting circuitry. The lenses remain in normally transparent state until a laser ray is sensed by the detector which energizes the lenses into a state of opacity by the interconnecting circuitry.

Therefore, it is an object of the invention to provide a personal laser protection device which is able to protect its user from laser radiation of a wavelength which is not known in advance.

### SUMMARY OF INVENTION

The object is solved by a device according to claim 1.

Further embodiments of the invention are described in the depending claims, the description and the appending drawings.

Where in the following the word invention is used and/or features are presented as optional this should be interpreted in such a way that protection is sought for the invention as claimed.

According to the invention, a personal laser protection device is disclosed. The laser protection device comprises at least fixing means being adapted to be worn on the user's head. On said fixing means, at least any of at least one protective filter and/or at least one laser beam entrance and/or at least one alarm system is attached. The laser beam entrance is coupled to at least one laser detection device.

The laser detection device is adapted to detect incident laser radiation. The laser detection device distinguishes incident laser radiation from other high intensity light sources such as direct sun light or an electric arc discharge. The laser detection device may be an integrated part of the personal laser protection device, i.e. the laser detection device may be arranged inside or may be attached to the fixing means. In other embodiments of the invention, the laser protection device may comprise its own housing so that it may be carried by the user on his belt or inside a pocket of his clothing. In these embodiments, the laser entrance and the laser detection device may be coupled by an optical fiber. In still another embodiment, the laser detection device may comprise a plurality of subcomponents which may be coupled by means of an optical, a wireless or an electrical communication line, e.g. optical components may be attached to the fixing means and electrical components may be worn in a pocket of the user's clothing.

Furthermore, the personal laser protection device may comprise a protective filter and/or an alarm system. The at least one protective filter is adapted to be arranged between a user's eye and a source of laser radiation. The protective filter is switchable from at least one first state to at least one second state, wherein incident radiation is attenuated at least partly on passing said protective filter in the second state. Incident radiation is not or at least less attenuated when passing said protective filter in the first state, thereby affecting the view of the user to a lesser extend. When operating the laser protection device, the protective filter may be switched from the first state to the second state if the laser detection device identifies laser light entering the laser beam entrance. After switching the protective filter to its second state, the incident laser radiation is at least attenuated or completely blocked so that the eyes of the user are not harmed or at least to a lesser extend. In some embodiments, switching of the protective filter may be wavelength selective or be carried out on a partial area of the filter only. In some embodiments of the invention, the protective filter may comprise a plurality of states blocking an increasing number of wavelengths, having increasing attenuation of transmitted intensity or blocking an increasing partial area of the filter. This allows adjustment of the filter to the incident radiation.

Aditionally to the protective filter, an alarm system is provided. The alarm system is adapted to emit an individually perceivable alarm. The alarm may comprise an acoustic alarm and/or a visual alarm. The alarm may warn a user from incident laser radiation. Furthermore, the alarm may warn a user before the protective filter is switched to the second state. This may prevent the user from being surprised by the protective filter which may affect his view.

The protective filter may be adapted to prevent a predefinable wavelength and/or a predefinable range of wavelengths to enter the user's eye. In some embodiments of the invention, the wavelength and/or the range of wavelengths may be selected from 0.4 - 1.4 µm, as such radiation will be focused on a user's eye and is capable of damaging the retina irreversibly. In other embodiments of the invention, the protective filter may attenuate the intensity of the spectrum for an equal amount. In still another embodiment of the invention, the protective filter may block all incident radiation.

The protective filter may be adapted to at least partly reflect incident radiation so that the reflected light can not enter the user's eye. In other embodiments of the invention, the protective filter may absorb at least a predefinable portion of the electromagnetic spectrum of incident radiation, thereby heating the protective filter but preventing the radiation to leave the protective filter and entering the user's eye.

In some embodiments of the invention, the protective filter may comprise a movable shutter. The shutter may be made from a metal or an alloy or a polymer material. The shutter may be closed by means of an electric motor, a piezo drive or a solenoid. A shutter may provide an easy and secure way to protect the user of the device from incident radiation by completely blocking the whole visible spectrum.

In some embodiments of the invention, the protective filter may comprise any of an electro-optical light modulator or a photochromic layer or a gasochromic layer or an electrochromic layer or a liquid crystal light modulator or a thermochromic filter. These filter elements are easily controllable by an electric signal which may be provided by a control unit or the laser detection device. Furthermore, the operating reliability may be increased as no moving parts are involved.

To detect laser radiation and to distinguish incident laser radiation from other high intensity light sources, the laser detection device may comprise means for determining a spectral distribution of incident light. As laser light is monochromatic or has a very narrow spectral distribution, an increasing intensity of a single wavelength may provide a reliable signal for emitting a warning or for switching the protective filter from its first state to its second state. In some embodiments of the invention, a plurality of spectral distributions may be stored inside the laser detection device so that the actual measured spectrum may be compared to a stored spectrum in order to determine the sources of electromagnetic radiation in the surroundings of the user. This may prevent a false alert which may be caused by gas discharge lamps or light emitting diodes having a narrow spectral distribution but a lower intensity.

In some embodiments of the invention the laser detection device may comprise means for determining the polarization state. Many different types of laser sources emit linearly polarized light. In contrast, incandescent lamps, sunlight, gas discharge lamps or other non-hazardous light sources emit unpolarized light. Therefore, determining the polarization state of incident radiation may provide a reliable signal for controlling the protective filter and/or the alarm system.

As the coherence length is defined by the speed of light divided by the spectral width of the radiation, a laser beam with narrow spectral distribution comprises a very large coherence length. Therefore, in some embodiments of the invention, the laser detection device may comprise means for determining a spatial coherence and/or means for determining a temporal coherence of incident light. In some embodiments, the laser detection device may comprise means for determining Speckle interferences and/or an interferometer. If coherent light is detected, the protective filter may be operated to switch from its first state to the second state and/or an alarm may be emitted.

In some embodiments of the invention, the laser detection device may comprise an integrated optical component. An integrated optical component may comprise optical components and optionally electrical components on a single substrate or a plurality of substrates which are combined by adhesive or a bonding process. An optical component may be selected from the group comprising lenses, waveguides, or mirrors and an electrical component may be selected from the group comprising photodiodes, amplifiers, triggers, output drivers or any other known elements. This will result in a very reliable yet compact construction of the laser detection device, so that the laser detection device may be easily integrated into the fixing means and the personal laser protection device will allow a reliable operation even in a harsh environment.

In some embodiments of the invention, a plurality of laser beam entrances may be arranged on said fixing means. This may allow verification of laser signals so that malfunction of the device may be reduced. In other embodiments of the invention, a plurality of laser beam entrances will allow for a spatial resolution of incident laser radiation. Therefore, the user may be informed about the direction the radiation is coming from or the protective filter may be operated to selectively block light from a predetermined dihedral angle.

In some embodiments of the invention said fixing means may comprise any of a spectacle frame or a goggle or a helmet or a headband. A spectacle frame allows the device to be very light weight, thereby increasing comfort. If said fixing means have the form of a helmet, the device may be integrated in equipment that the user usually wears, e.g. if the user is a soldier or a policemen. A headband may be used to attach earphones and/or a microphone. Thus, the headband may be adapted to additionally carry the proposed device for laser protection. Such an embodiment may be in particular useful for pilots.

In some embodiments of the invention, the device may comprise further a communication unit being adapted to send and/or receive information concerning incident laser radiation. If, for example, a single policeman out of the group is dazzled by laser light, his personal protection device will notice the narrow band, high intensity and coherent light. A warning will be issued to the policeman and the protective filter will be switched to reduce the intensity of laser light reaching his eyes. At the same time, a wireless communication signal is sent out which can be received by the other policemen of the group. Their respected protection devices issue a warning. Therefore, they are also aware of the high intensity light before the beam actually hits them. These policemen now are able to prosecute the offender as their view is not affected by the protective filter.

### BRIEF DESCRIPTION OF DRAWINGS

The foregoing aspects and many of the attended advantages of this invention will become more readily appreciated as the same becomes better understood by reference to the following detailed description, when taken in conjunction with the accompanying drawings, wherein:
- Fig. 1: shows a first embodiment of a device according to the invention.
- Fig. 2: shows a second embodiment of a device according to the invention.
- Fig. 3: shows a first embodiment of a laser detection device.
- Fig. 4: shows a first embodiment of a protective filter in a first state.
- Fig. 5: shows an embodiment of a protective filter in a second state.
- Fig. 6: shows a second embodiment of a laser detection device.
- Figs. 7 and 8: illustrate the working principle of the second embodiment of the laser detection device.
- Fig. 9: shows a third embodiment of a laser detection device.
- Fig. 10: shows a fourth embodiment of a laser detection device.
- Fig. 11: shows an example of an integrated optical component.
- Fig. 12: shows a block diagram of the personal laser protection device according to the invention.

### DETAILED DESCRIPTION

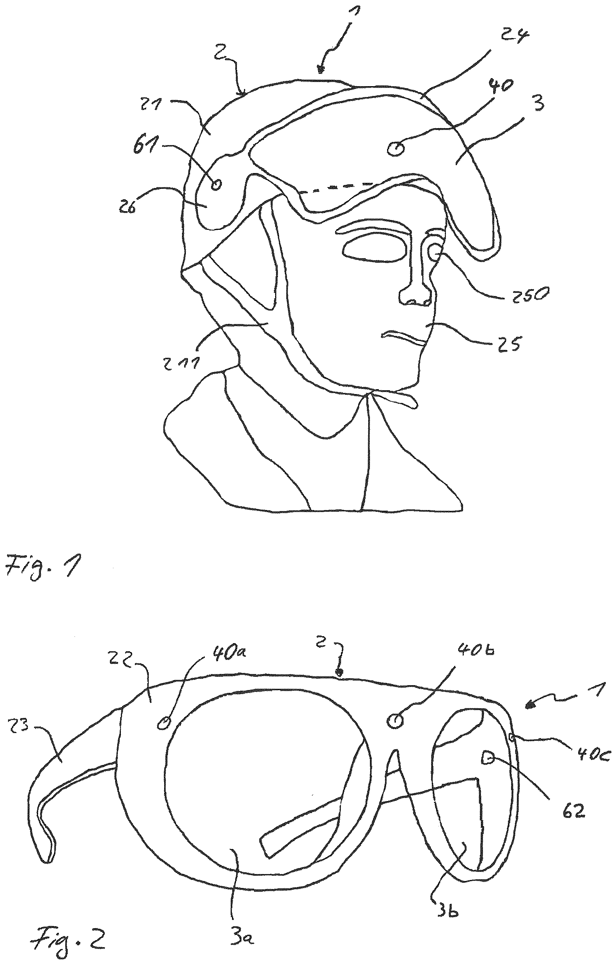

Fig. 1 shows a first embodiment of a laser detection device according to the invention. The personal laser detection device 1 comprises fixing means 2 being adapted to be worn on the head 25 of a user. In the first embodiment of the invention, the fixing means comprise a helmet 21. The helmet 21 may cover at least a part of the user's head 25.

In some embodiments of the invention, the helmet 21 may also cover the ears of the user. In these embodiments, the helmet 21 may carry earphones or loudspeakers 61 which are intended to emit an audible alarm.

The helmet 21 may comprise a strap 211 which is intended to fix the helmet 21 on the user's head 25. The helmet 21 may be adapted to protect the user from falling or thrown objects and/or protect the user's head 25 if the user is subject to an impact on a solid body. Therefore, the helmet 21 may comprise a hard shell and/or a padding as known from the art.

Furthermore, the helmet 21 is adapted to carry at least one protective filter 3. The filter 3 is arranged in a frame 24. The frame 24 may be made from a metal, an alloy or a plastics material. In some embodiments of the invention, the frame 24 may be made from fiber reinforced plastics in order to also give some mechanical protection for the user's face.

In some embodiments of the invention the frame 24 is supported in hinges 26 which allow to deviate the protective filter 3 from a first position to a second position. In the first position, the protective filter 3 may cover a part of the helmet 21. In this position, the eyes 250 of the user 25 may not be covered by the protective filter 3. In the second position, the protective filter 3 may be arranged between a user's eye 250 and a source of laser radiation. Therefore, the first position may be referred to as the open position and the second position may be referred to as the closed position. However, it has to be noted that hinges 26 are completely optional and may be omitted in other embodiments of the invention.

The protective filter 3 is switchable from at least one first state to at least one second state, wherein incident radiation is attenuated at least partly on passing said protective filter in the second state. This means, that switching the protective filter 3 to the second state will absorb or reflect at least a part of the electromagnetic spectrum. In other embodiments of the invention, the protective filter may be adapted to absorb or reflect all of the electromagnetic spectrum. In some embodiments of the invention, at least a partial area of the total surface of the protective filter 3 may be switched into a second state. In other embodiments of the invention, the full surface area may be switched from a first state into a second state when incident laser radiation is detected. Thus, in the second state of the protective filter 3, the eyes 250 of the user are protected from incident laser radiation. However, in the first state of the protective filter 3, the user's view and optical orientation may be less affected by the presence of the protective filter 3.

In order to determine which state of the protective filter is appropriate, the fixing means 2 are equipped with a laser beam entrance 40. The laser beam entrance 40 is adapted to receive incoming light and forward this optical signal to at least one laser detection device. The laser detection device will determine if incoming light comprises harmful laser radiation and issue any of an individually perceivable alarm and/or a switching signal to switch the protective filter 3 from at least one first state to at least one second state or vice versa.

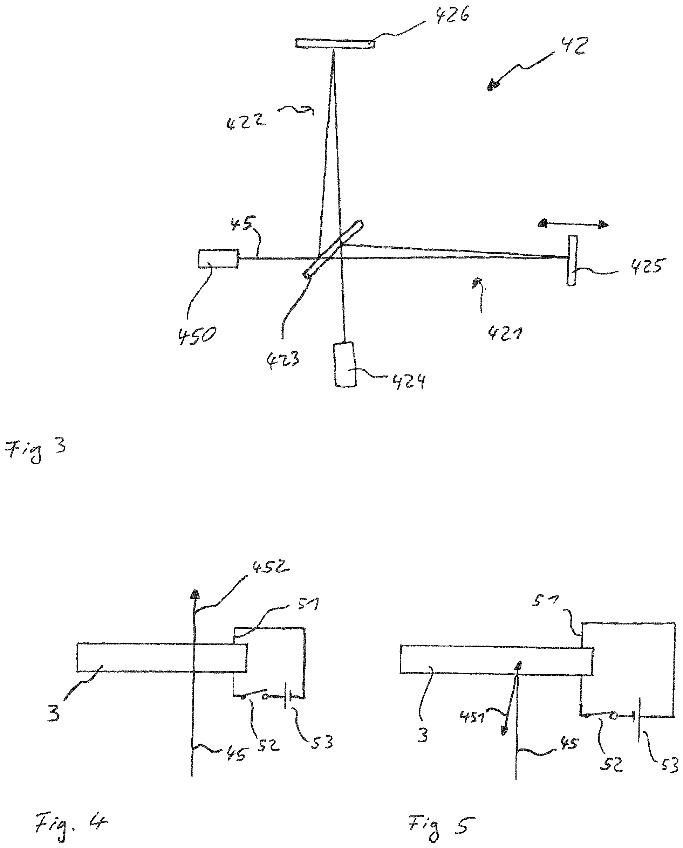

The laser detection device may be fixed on or in the helmet 21 in order to provide the compact laser protection device 1. In other embodiments of the invention, the laser detection device or parts of it may be coupled to the personal laser detection device 1 by means of a cable or an optical fiber. In this case, the laser detection device may be carried by the user in his pocket or on a belt. Different embodiments of the laser detection device are detailed with respect to Figs. 3 and 6 to 10.

The protective filter 3 may comprise any of a mechanical shutter, an electro-optical light modulator or a photochromic layer, a gasochromic layer, an electrochromic layer or a liquid-crystal light modulator or a thermochromic filter. All these devices can be controlled by an electrical signal generated by the laser detection device. In other embodiments of the invention, the user may be able to switch the protective filter 3 by a switch or by a voice control system if the state assigned automatically by the laser detection device is not appropriate for a given situation.

Fig. 2 shows a second embodiment of the invention. Same parts are assigned the same reference numbers, so that the following description is restricted to the main differences. A personal laser detection device according to the second embodiment of the invention comprises fixing means 2. The fixing means 2 are embodied as a spectacle frame 22, which may be fixed on a user's head by means of temples 23. Thus, the personal laser protection device 1 according to the second embodiment may be worn by a user like sunglasses known from the art.

The laser protection device 1 comprises two protective filters 3. Each protective filter is designed to be arranged between one eye 250 of the user and the source of laser light. In some embodiments of the invention, both protective filters may be switched independently, depending on the direction where dazzling laser light is arriving from.

The laser protection device comprises further three laser beam entrances 40a, 40b and 40c. A plurality of laser beam entrances 40 may allow a precise determination of the direction of incoming laser light. Furthermore, a plurality of laser beam entrances and/or a plurality of laser detection devices may improve security as the electronics and optics is redundant and the device may be fully operational even if parts of the electronic or optical system may fail.

It should be clear that the invention is not limited to the exact number of one or three laser entrances 40. In other embodiments of the invention, the number and location of the laser beam entrances 40 may be different. For example, laser beam entrances 40 may surround the protective filter 3 and allow a precise determination of the direction of incoming laser light.

Furthermore, Fig. 2 shows an emitting diode 62 which is arranged at the inside of the spectacle frame 2. The light-emitting diode 62 may be used to emit an optical alarm to the user. The light-emitting diode 62 may be adapted to emit light in the direction of the protective filter 3 which is partly reflected at the inside of the protective filter 3 in order to reach the eye 250 of the user. In some embodiments of the invention, two light-emitting diodes 62 may be arranged in the spectacle frame 22 and allow emitting separate warnings to the right and left eyes of the user. Thus, the user may see an optical alarm indicating the direction of incoming laser light. In other embodiments of the invention, an optical alarm may be generated by other light emitting means such as an OLED, a dot-matrix display, a liquid-crystal-display or other means known from the art.

In some embodiments of the invention the light-emitting diode 62 may be used to emit an optical warning to the user prior to switch the protective filter from the first state to the second state.

It should be clear from the description that the alarm systems 61 and 62 may be combined with any embodiment of the invention. The embodiment according to Fig. 1 therefore may comprise a light-emitting diode 62. Furthermore, the embodiment according to Fig. 2 may comprise additionally or exclusively an earphone to emit an audible warning. Earphones may be carried by the user using a headband and may be used additionally for radio communication or they may be included into the temples 23 as known from hearing aids.

With respect to Figs. 4 and 5, the function of the laser protective filter 3 is shown. The protective filter 3 comprises any of an electrochromic or a thermochromic layer, i.e. the absorption and/or reflection of the protective filter is adjusted by an electric field or a certain temperature. In other embodiments of the invention, the protective filter 3 may comprise a movable shutter or a liquid crystal light modulator. In any embodiments of the invention, the absorption and/or reflection of the protective filter 3 may be adjusted by means of an electrical signal. The electrical signal is transported to the protective filter 3 by means of vias 51.

Figs. 4 and 5 show a simple example to illustrate the principle of operation of the protective filter 3. Other working embodiments of the invention may comprise a more complex circuitry.

The protective filter 3 is controlled by means of an electrical signal which is generated by a voltage source and a switch. Figure 4 shows the protective filter in its first state and figure 5 shows the protective filter in its second state.

As can be seen in Fig. 4, incoming laser light 45 is transmitted through the protective filter 3. Therefore, light 452 is able to enter the eye of a user. This is the first state of the switchable protective filter 3. No current flows through the protective filter 3 and/or no electric voltage is present.

Closing the switch 52 allows a voltage and/or a current generated by a battery 53 to act on the protective filter. This will switch the filter in its second state. Incoming light 45 is at least partially reflected as beam 451 or absorbed inside the protective filter 3. Therefore, the light may not enter the eye of a user.

Fig. 3 shows a first embodiment of a laser detection device. The laser detection device comprises means 42 for determining a temporal coherence of incident light. A laser beam 45 from a laser source 450 may be coupled to the laser beam input 40. The laser light 45 then reaches a beam splitter 423. One part of the laser light is transmitted to form the first optical path 421. Another part of the light is reflected to form a second optical path 422. Both optical paths 421 and 422 are limited by a respective mirror 426 and 425. The reflected light is sent back to the beam splitter 423. Light reaching the detector 424 interferes so that an interference pattern or a changing intensity may be detected. The detector 424 may comprise any of a CCD line detector, a two-dimensional CCD detector or just a photodiode.

If a CCD detector is used, an interference pattern may be identified clearly. An occurrence of an interference pattern indicates that coherent light is entering the laser detection device. If no interference pattern is detected, only incoherent light such as sunlight is entering the laser detection device.

In some embodiments of the invention, at least one mirror such as mirror 225 may be moving in order to alter the path length of the optical path 221 with time. In this case, a simple photodiode may be used as detector 424. If incoherent light is entering the laser detection device, a constant signal or a DC voltage is generated by the photodiode. If coherent light enters the device, the photodiode will additionally produce an alternating current which may be detected easily.

Fig. 3 shows a Michelson interferometer as an exemplary embodiment of the invention. It should be clear that other types of interferometers may be used as well, just like Fabry-Pérot interferometers, Mach-Zehnder interferometers, Twyman-Green interferometers, Rayleigh interferometers, Sagnac interferometers or Fizeau interferometers.

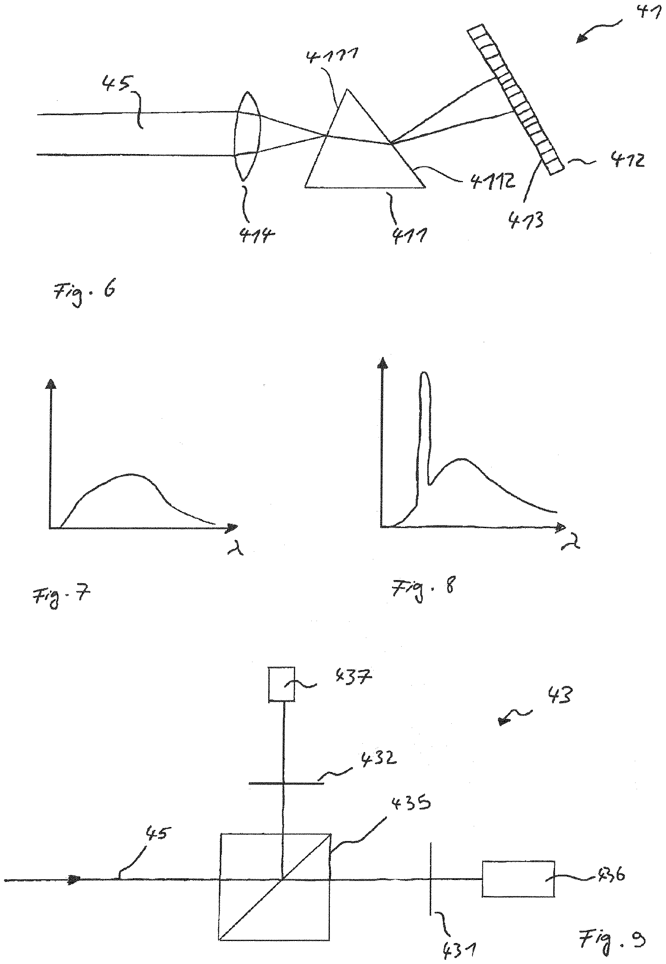

Fig. 6 shows a second embodiment of a laser detection device 4. The laser detection device according to Fig. 6 comprises means 41 for determining a spectral distribution of incident light. The principle of operation is explained with respect to Figs. 7 and 8.

Light 45 entering the laser detection device from the laser beam entrance 40 is coupled to a prism 411. Coupling of the light to the entrance 4111 of the prism 411 may be improved by an optional optics 414. In other embodiments of the invention, any other dispersive element known from the art may be used such as a grating, a volume holographic grating or an acoustic-optical modulator.

The optics 414 is shown in Fig. 6 as a simple focusing lens. It should be clear that in other embodiments of the invention, a more complex optical system may be used which may comprise a plurality of focusing or defocusing lenses. The light entering the prism 411 from one side 4111 is transmitted through the prism and leaves the prism at the outlet 4112. The light from the output is coupled to the detector 412. It should be clear that an optional optical system may be used between the outlet 4112 of the prism 411 and the detector 412 as well.

The prism 411 is made from a dispersive material and therefore divides the light into its spectral components. This means that light of different wavelengths is depicted on different locations of the detector 412. The detector 412 may comprise a plurality of cells 413. In some embodiments of the invention, the detector 412 may comprise a linear CCD sensor or a photodiode array.

The detector signal of the detector 412 is shown in Figs. 7 and 8. Fig. 7 shows the wavelength or the number of the respective cell 413 on the abscissa and the relative intensity at the ordinate. The example shown in Fig. 7 may be true for a source of white light such as an incandescent lamp or sunlight. The signal shown in Fig. 7 would cause the protective filter to be in its first state.

When laser light enters the device, a spectrum as shown in Fig. 8 may be achieved. Fig. 8 shows basically the same broad distribution perceived as white light as Fig. 7. However, a single wavelength with excessive intensity is additionally present. This signal indicates clearly harmful monochromatic laser radiation. Therefore, the protective filter may be switched to its second state to protect the user.

The third embodiment of the laser detection device 4 is shown with respect to Fig. 9. Fig. 9 details one embodiment of means 43 for determining a polarization state. Incoming light 45 is coupled to a beam splitter 435. The light leaving the beam splitter is directed to different polarization filters 431 and 432 having different orientations. In some embodiments of the invention, the polarization filter 431 and 432 may comprise linear polarization filters having different orientation. In some embodiments of the invention, the orientation may be approximately 90° to each other. In other embodiments of the invention, the filters 431 and 432 may comprise circular polarization filters with different orientation, i.e. one polarization filter may have a left orientation and the other polarization filter may have a right orientation.

The light transmitted by the polarization filters 431 and 432 is detected by detectors 436 and 437, respectively. Detectors 436 and 437 may comprise a photodiode.

If unpolarized light enters the device 43, approximately the same intensity is detected in both photodiodes 436 and 437. If polarized laser light enters the device, the signal will become asymmetric as one polarizer will block a part of the intensity. Therefore, evaluating the detector signals of the photodiodes 436 and 437 by a differential amplifier will easily detect harmful dazzling laser radiation.

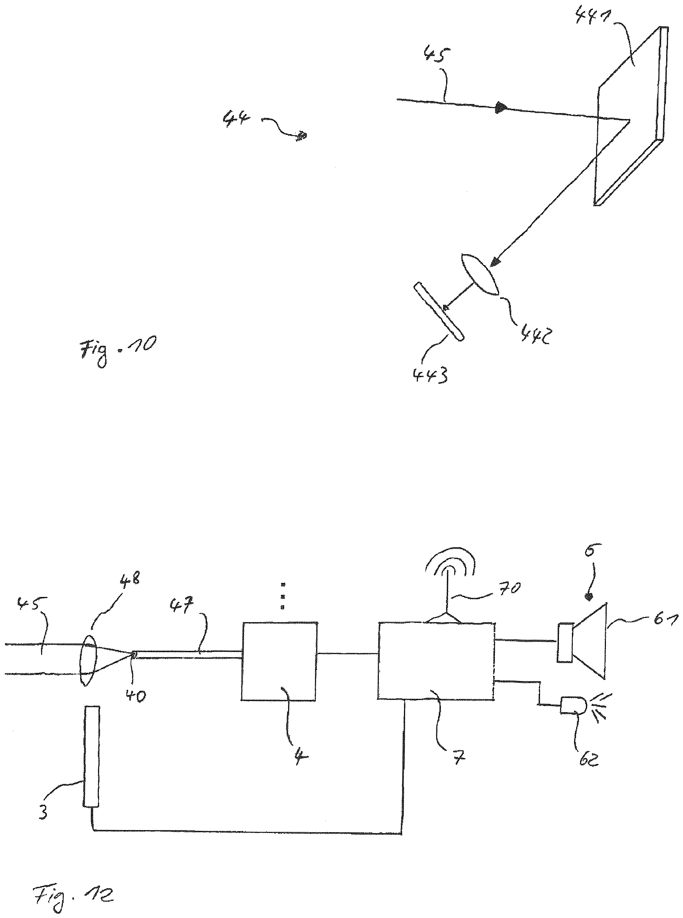

A fourth embodiment of a laser detection device is explained with respect to Fig. 10. The laser detection device 4 according to the fourth embodiment comprises means 44 for determining a spatial coherence of incident light 45. Incident light 45 is brought to a screen 421. The incident light 45 may be focused by means of at least one optical element like a lens. The light reflected on the screen 441 is detected by a two-dimensional optical detector 443. In some embodiments of the invention, the detector 443 may comprise a CCD chip. In order to generate an image on the CCD chip 443, an optics 442 may be used. In some embodiments of the invention, the optics 442 may be adapted to generate a blurred image on the surface of the detector 443.

On operation, incident light being reflected on the screen 441 will produce a constant signal in each cell of the detector 443 if incoherent light is present. Even if the screen 441 is not polished, the surface will have randomly orientated partial areas. Therefore, incident coherent radiation will cause Speckle interferences in the reflected beam. These speckle interferences will be detected on the detector 443 as light spots of increased intensity. This light pattern will vary randomly in time. Even if the image generated by the optics 442 is blurred, the speckle interferences will be depicted as a sharp image on the surface of the detector 443. Therefore, any signal being different from a constant intensity over a plurality of pixels will indicate the presence of harmful laser radiation.

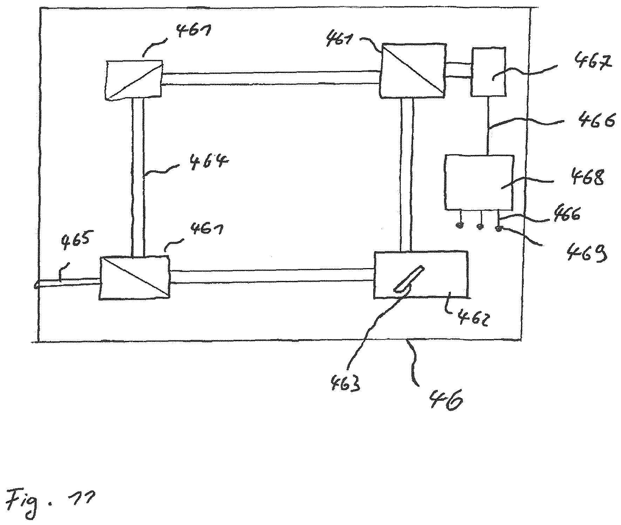

Fig. 11 shows an exemplary embodiment of an integrated optical component 46. The integrated optical component 46 may be arranged on a substrate made of any of a glass, a polymer or a semiconductor, e.g. silicon. The substrate 46 may be adapted to support a plurality of optical and/or electronic devices. Such a fully integrated configuration may increase the reliability of the laser detection device.

As an exemplary embodiment, Fig. 11 shows a Fabry-Pérot interferometer which is composed of a plurality of fixed mirrors 461 which may act as beam splitters. Furthermore, a moveable mirror 463 is arranged in a recess 462. The recess 462 and the movable mirror may be made by photolithography and etching techniques known from the art or by conventional micromechanical techniques such as micromilling. The mirror 463 may be moved by a piezo drive or capacitively coupling of an alternating electrical signal. The optical components may be connected to each other by means of integrated waveguides 464. The waveguides 464 may be obtainable by femtosecond laser radiation. Such laser radiation may be used to modify the material of the substrate in order to get a sufficient difference in the refractive index. In the same way, an input waveguide 465 may be obtained.

If the substrate comprises a semiconducting material, a detector 467 may be monolithically integrated onto the substrate. The detector 467 may comprise a photodiode. Furthermore, electronic devices 468 may be integrated onto the substrate. The electronic devices may comprise transistors, resistors, capacitors, amplifiers, A/D converters or other electronic devices known from the art. Furthermore, interconnects 466 between the detector 467 and the electronic devices 468 may be provided by at least one metallization layer which may be structured by photolithography. Electric signals may be connected by contact pads 469.

It has to be noted, that the foregoing description does not limit the invention to the described exemplary embodiment. In other embodiments of the invention, other optical and/or electronic devices may be integrated onto the substrate. The embodiment of the invention in form of an integrated optical component allows a very compact and reliable design which may be easily integrated into the fixing means 2.

Fig. 12 explains a block diagram of the invention. Fig. 12 shows the laser beam entrance 40. An optional optical device 48 may be used to focus incoming laser light 45 onto the laser beam entrances 40 and/or to match the phase space volume to the acceptance of the optical fiber 47. The optical fiber 47 is coupled to a laser detection device 4 as detailed above. If a plurality of laser beam entrances 40 is present, the light coupled to the individual laser beam entrances may be coupled to a single laser detection device 4 by means of a multiplexer or a plurality of laser detection devices 4 may be present which are electrically coupled to a central processing unit 7.

The central processing unit 7 comprises a decision making unit which decides on the appropriate state of the protective filter 3. The appropriate state is indicated by an electrical signal and supplied to the protective filter 3.

The decision making unit may comprise a microcontroller or a microprocessor and a software which is adapted to analyze the electrical signals delivered by the laser detection device 4. Analyzing the signals may comprise a comparison between the values actually delivered with stored values for different cases of operation. If a known pattern is recognized, the appropriate state may be selected from a plurality of stored possibilities. In other embodiments of the invention, the decision making unit may comprise a fuzzy logic or a neural network to make an appropriate decision about the presence or non-presence of harmful laser radiation.

Furthermore, an optional alarm system 6 may be present which is adapted to emit an individually perceivable alarm. The alarm may be emitted acoustically by means of a headphone or a speaker 61. In other embodiments of the invention, the alarm may be emitted as visible signal by means of a light-emitting diode 62. It has to be noted that any of the alarm system 6 or the protective filter 3 are optional. The personal laser protection device according to the invention may either warn the user from harmful radiation so that the user may view in another direction. In other embodiments, the laser protection device may protect the user by switching a protective filter. In still another embodiment of the invention, the laser protection device may warn the user and switch the protective filter.

Furthermore, an optional communication unit 70 may be present. The communication unit 70 may setup a bidirectional radio communication so that the laser protection devices from the group of persons are able to communicate to each other. This may allow other users to be warned if one user is hit by a laser beam. In other embodiments of the invention, all protective filters of the group may be switched to an opaque state if one user is hit by a laser beam. In still another embodiment, the laser protection devices may allow for manual operation by means of a switch and the manual operation of a single user may be communicated to other users.

The illustrative embodiments and modifications thereto described herein above are merely exemplary. It is understood that other modifications to this illustrative embodiments will regularly occur to persons of ordinary skill in the art. All such modifications and variations are deemed to be within the scope of the present invention as will be defined in the accompanying claims. The above-described embodiments may be used alone or combined for use.

## Claims

1. Personal laser protection device (1) comprising:
Fixing means (2) being adapted to be worn on a user's head (25) and further being adapted to carry at least any of at least one protective filter (3) and at least one laser beam entrance (40) and at least one optional alarm system (6), wherein
the laser beam entrance (40) is coupled to at least one laser detection device (4) being adapted to detect incident laser radiation (45) and the laser detection device (4) being adapted to distinguish incident laser radiation from other high intensity light sources, and
the protective filter (3) is adapted to be arranged between a user's eye (250) and a source of laser radiation (450) and is switchable from at least one first state to at least one second state, wherein incident radiation (45) is attenuated at least partly on switching said protective filter in the second state, and
the alarm system (6) is adapted to emit an individually perceivable alarm.

2. Device according to claim 1, wherein the protective filter (3) is adapted to at least partly absorb at least a predefinable portion of the electromagnetic spectrum of incident radiation (45) and/or wherein the protective filter (3) is adapted to at least partly reflect at least a predefinable portion of the electromagnetic spectrum of incident radiation (45).

3. Device according to any of claims 1 or 2, wherein the protective filter (3) comprises any of a movable shutter or an electro-optical light modulator or a photochromic layer or a gasochromic layer or an electrochromic layer or a liquid-crystal light modulator or a thermochromic filter and/or wherein the movable shutter is actuated by any of an electric motor, a piezo drive or a solenoid.

4. Device according to any of claims 1 to 3, wherein the laser detection device (4) comprises any of means (41) for determining a spectral distribution of incident light or means (43) for determining a polarization state or means (44) for determining a spatial coherence or means (42) for determining a temporal coherence of incident light.

5. Device according to any of claims 1 to 4, wherein the laser detection device (4) comprises any of a prism (411) or an optical grating having an input (4111) and an output (4112), wherein incident light (45) can be coupled to the input (4111) and light from the output (4112) may be coupled to a CCD line sensor (412).

6. Device according to any of claims 1 to 5, wherein the laser detection device (4) comprises at least one polarization filter (431, 432) having an input and an output, wherein incident light can be coupled to the input and light from the output may be coupled to at least one photodetector (437, 436).

7. Device according to any of claims 1 to 6, wherein the laser detection device (4) comprises
an interferometer having at least a first optical path (421) with a first optical path length and
a second optical path (422) with a second optical path length and
at least one beam splitter (423) with at least one input and at least two outputs, wherein
when operating the device, incident light (45) is coupled to the input of the beam splitter (423) and the beam splitter is adapted to supply said light to the first and second optical paths (421, 422), and wherein the light from said first and second optical path (421, 422) may be brought to interference before being detected by a photodetector (424), wherein
at least one optical path length is variable in time.

8. Device according to any of claims 1 to 7, wherein the laser detection device (4) comprises
a screen (441) being adapted to receive incident light (45), and
an optics (442) being adapted to image at least a part of said screen to a sensor (443) having a spatial resolution.

9. Device according to claim 8, wherein the image on the sensor is blurred.

10. Device according to any of claims 1 to 9, wherein the laser detection device (4) comprises an integrated optical component (46).

11. Device according to any of claims 1 to 10, wherein said fixing means (2) comprises any of a spectacle frame (22, 23) or a helmet (21) or a head band.

12. Device according to any of claims 1 to 11, wherein the laser beam entrance (40) is coupled to at least one laser detection device (4) by means of an optical fiber (47).

13. Device according to any of claims 1 to 12, wherein the alarm system (6) comprises any of a light emitting diode (62) or a loudspeaker (61) and/or wherein the alarm system (6) is adapted to emit an alarm before the protective filter (3) is actuated.

14. Device according to claim 13, wherein the alarm system (6) is adapted to indicate any of a direction or an intensity of incident radiation (45).

15. Device according to any of claims 1 to 14, comprising further a communication unit (70) being adapted to send and/or receive information concerning incident laser radiation (45).

## Patentansprüche

1. Persönliche Laserschutzeinrichtung (1), umfassend:
ein Fixierungsmittel (2), das dazu ausgelegt ist, an dem Kopf (25) eines Benutzers getragen zu werden, und das weiterhin zum Tragen mindestens eines Schutzfilters (3) und/oder mindestens eines Laserstrahleintritts (40) und/oder mindestens eines fakultativen Alarmsystems (6) ausgelegt ist, wobei
der Laserstrahleintritt (40) mit mindestens einer Laserdetektionseinrichtung (4) gekoppelt ist, die dazu ausgelegt ist, einfallende Laserstrahlung (45) zu detektieren, und wobei die Laserdetektionseinrichtung (4) dazu ausgelegt ist, einfallende Laserstrahlung von anderen Lichtquellen hoher Intensität zu unterscheiden, und
das Schutzfilter (3) dazu ausgelegt ist, zwischen einem Auge (250) eines Benutzers und einer Laserstrahlungsquelle (450) angeordnet zu werden, und von mindestens einem ersten Zustand in mindestens einen zweiten Zustand schaltbar ist, wobei einfallende Strahlung (45) zumindest zum Teil beim Schalten des Schutzfilters in den zweiten Zustand gedämpft wird, und
das Alarmsystem (6) dazu ausgelegt ist, einen individuell wahrnehmbaren Alarm auszugeben.

2. Einrichtung nach Anspruch 1, wobei das Schutzfilter (3) dazu ausgelegt ist, zumindest einen vordefinierten Anteil des elektromagnetischen Spektrums von einfallender Strahlung (45) zumindest teilweise zu absorbieren, und/oder wobei das Schutzfilter (3) dazu ausgelegt ist, zumindest einen vordefinierten Anteil des elektromagnetischen Spektrums von einfallender Strahlung (45) zumindest teilweise zu reflektieren.

3. Einrichtung nach einem der Ansprüche 1 oder 2, wobei das Schutzfilter (3) eines der Folgenden umfasst:
einen beweglichen Verschluss oder einen elektrooptischen Lichtmodulator oder eine photochrome Schicht oder eine gasochrome Schicht oder eine elektrochrome Schicht oder einen Flüssigkristalllichtmodulator oder ein thermochromisches Filter, und/oder wobei der bewegliche Verschluss von einem elektrischen Motor, einem Piezoantrieb oder einem Solenoid angetrieben wird.

4. Einrichtung nach einem der Ansprüche 1 bis 3, wobei die Laserdetektionseinrichtung (4) eines der Folgenden umfasst: Mittel (41) zum Bestimmen einer Spektralverteilung von einfallendem Licht oder Mittel (43) zum Bestimmen eines Polarisationszustands oder Mittel (44) zum Bestimmen einer räumlichen Kohärenz oder Mittel (42) zum Bestimmen einer zeitlichen Kohärenz von einfallendem Licht.

5. Einrichtung nach einem der Ansprüche 1 bis 4, wobei die Laserdetektionseinrichtung (4) eines der Folgenden umfasst: ein Prisma (411) oder ein optisches Gitter mit einem Eingang (4111) und einem Ausgang (4112), wobei einfallendes Licht (45) in den Eingang (4111) eingekoppelt werden kann und Licht aus dem Ausgang (4112) in einen CCD-Zeilensensor (412) eingekoppelt werden kann.

6. Einrichtung nach einem der Ansprüche 1 bis 5, wobei die Laserdetektionseinrichtung (4) mindestens ein Polarisationsfilter (431, 432) mit einem Eingang und einem Ausgang umfasst, wobei einfallendes Licht in den Eingang eingekoppelt werden kann und Licht aus dem Ausgang in mindestens einen Photodetektor (437, 436) eingekoppelt werden kann.

7. Einrichtung nach einem der Ansprüche 1 bis 6, wobei die Laserdetektionseinrichtung (4) Folgendes umfasst:
ein Interferometer mit mindestens einem ersten optischen Weg (421) mit einer ersten optischen Weglänge und
einen zweiten optischen Weg (422) mit einer zweiten optischen Weglänge und
mindestens einen Strahlteiler (423) mit mindestens einem Eingang und mindestens zwei Ausgängen, wobei
bei Betrieb der Einrichtung einfallendes Licht (45) in den Eingang des Strahlteilers (423) eingekoppelt wird und der Strahlteiler dazu ausgelegt ist, das Licht dem ersten und zweiten optischen Weg (421, 422) zuzuführen, und wobei das Licht aus dem ersten und zweiten optischen Weg (421, 422) zur Interferenz gebracht werden kann, bevor es von einem Photodetektor (424) detektiert wird, wobei
zumindest eine optische Weglänge zeitlich variabel ist.

8. Einrichtung nach einem der Ansprüche 1 bis 7, wobei die Laserdetektionseinrichtung (4) folgendes umfasst:
einen Schirm (441), der zur Aufnahme von einfallendem Licht (45) ausgelegt ist, und
eine Optik (442), die zum Abbilden mindestens eines Teils des Schirms auf einen Sensor (443) mit einer räumlichen Auflösung ausgelegt ist.

9. Einrichtung nach Anspruch 8, wobei das Bild auf dem Sensor unscharf ist.

10. Einrichtung nach einem der Ansprüche 1 bis 9, wobei die Laserdetektionseinrichtung (4) eine integrierte optische Komponente (46) umfasst.

11. Einrichtung nach einem der Ansprüche 1 bis 10, wobei das Fixierungsmittel (2) eine Brillenfassung (22, 23) oder einen Helm (21) oder ein Kopfband umfasst.

12. Einrichtung nach einem der Ansprüche 1 bis 11, wobei der Laserstrahleintritt (40) mittels einer optischen Faser (47) mit mindestens einer Laserdetektionseinrichtung (4) gekoppelt ist.

13. Einrichtung nach einem der Ansprüche 1 bis 12, wobei das Alarmsystem (6) eine Leuchtdiode (62) oder einen Lautsprecher (61) umfasst, und/oder wobei das Alarmsystem (6) dazu ausgelegt ist, einen Alarm auszugeben, bevor das Schutzfilter (3) betätigt wird.

14. Einrichtung nach Anspruch 13, wobei das Alarmsystem (6) dazu ausgelegt ist, eine Richtung oder eine Intensität der einfallenden Strahlung (45) anzugeben.

15. Einrichtung nach einem der Ansprüche 1 bis 14, ferner umfassend eine Kommunikationseinheit (70), die dazu ausgelegt ist, Informationen über die einfallende Laserstrahlung (45) zu senden und/oder zu empfangen.

## Revendications

1. Dispositif de protection anti-laser personnel (1) comprenant :
un moyen de fixation (2) qui est adapté à être porté sur la tête d'un utilisateur (25) et qui est en outre adapté à porter au moins un élément quelconque parmi au moins un filtre protecteur (3) et au moins une entrée de faisceau laser (40) et au moins un système d'alarme optionnel (6), dans lequel
l'entrée de faisceau laser (40) est couplée à au moins un dispositif de détection de laser (4) qui est adapté à détecter un rayonnement laser incident (45) et le dispositif de détection de laser (4) est adapté à distinguer un rayonnement laser incident vis-à-vis d'autres sources de lumière à forte intensité, et
le filtre protecteur (3) est adapté à être agencé entre un oeil (250) d'un utilisateur et une source de rayonnement laser (450) et est susceptible d'être commuté depuis au moins un premier état vers au moins un second état, de sorte que le rayonnement incident (45) est atténué au moins partiellement lors de la commutation dudit filtre protecteur dans le second état, et
le système d'alarme (6) est adapté à émettre une alarme individuellement perceptible.

2. Dispositif selon la revendication 1, dans lequel le filtre protecteur (3) est adapté à absorber au moins partiellement au moins une portion prédéfinissable du spectre électromagnétique du rayonnement incident (45) et/ou dans lequel le filtre protecteur (3) est adapté à au moins partiellement réfléchir au moins une portion prédéfinissable du spectre électromagnétique du rayonnement incident (45).

3. Dispositif selon l'une quelconque des revendications 1 et 2, dans lequel le filtre protecteur (3) comprend un élément quelconque parmi un obturateur mobile ou un modulateur de lumière électro-optique ou une couche photochromatique ou une couche gazochromatique ou une couche électrochromatique ou un modulateur de lumière à cristaux liquides ou un filtre thermochromatique et/ou dans lequel l'obturateur mobile est actionné par un moyen quelconque parmi un moteur électrique, un entraînement piézoélectrique ou un solénoïde.

4. Dispositif selon l'une quelconque des revendications 1 à 3, dans lequel le dispositif de détection de laser (4) comprend un moyen quelconque parmi un moyen (41) pour déterminer une distribution spectrale de la lumière incidente, ou un moyen (43) pour déterminer un état de polarisation, ou un moyen (44) pour déterminer une cohérence spatiale, ou un moyen (42) pour déterminer une cohérence temporelle de la lumière incidente.

5. Dispositif selon l'une quelconque des revendications 1 à 4, dans lequel le dispositif de détection de laser (4) comprend un élément quelconque parmi un prisme (411) ou un réseau optique ayant une entrée (4111) et une sortie (4112), dans lequel la lumière incidente (45) peut être couplée à l'entrée (4111) et la lumière venant de la sortie (4112) peut être couplée à un capteur en ligne à CCD (412).

6. Dispositif selon l'une quelconque des revendications 1 à 5, dans lequel le dispositif de détection de laser (4) comprend au moins un filtre de polarisation (431, 432) ayant une entrée et une sortie, dans lequel la lumière incidente peut être couplée à l'entrée et la lumière venant de la sortie peut être couplée à au moins un photodétecteur (437, 436).

7. Dispositif selon l'une quelconque des revendications 1 à 6, dans lequel le dispositif de détection de laser (4) comprend
un interféromètre ayant au moins un premier trajet optique (421) avec une première longueur de trajet optique, et
un second trajet optique (422) avec une seconde longueur de trajet optique, et
au moins un séparateur de faisceau (423) avec au moins une entrée et au moins de sortie, dans lequel
lors du fonctionnement du dispositif, la lumière incidente (45) est couplée à l'entrée du séparateur de faisceau (423) et le séparateur de faisceau est adapté à fournir ladite lumière au premier et au second trajet optique (421, 422), et dans lequel la lumière provenant dudit premier et dudit second trajet optique (421, 422) peut être amenée à interférer avant d'être détectée par un photodétecteur (424), dans lequel au moins une longueur de trajet optique est variable dans le temps.

8. Dispositif selon l'une quelconque des revendications 1 à 7, dans lequel le dispositif de détection de laser (4) comprend un écran (441) qui est adapté à recevoir la lumière incidente (45), et une unité optique (442) qui est adaptée à produire l'image d'au moins une partie dudit écran sur un capteur (443) ayant une résolution spatiale.

9. Dispositif selon la revendication 8, dans lequel l'image sur le capteur est floue.

10. Dispositif selon l'une quelconque des revendications 1 à 9, dans lequel le dispositif de détection de laser (4) comprend un composant optique intégré (46).

11. Dispositif selon l'une quelconque des revendications 1 à 10, dans lequel ledit moyen de fixation (2) comprend un moyen quelconque parmi une monture de lunettes (22, 23) ou un casque (21) ou un bandeau de tête.

12. Dispositif selon l'une quelconque des revendications 1 à 11, dans lequel l'entrée de faisceau laser (40) est couplée à au moins un dispositif de détection de laser (4) au moyen d'une fibre optique (47).

13. Dispositif selon l'une quelconque des revendications 1 à 12, dans lequel le système d'alarme (6) comprend un élément quelconque parmi une diode électroluminescente (62) ou un haut-parleur (61), et/ou dans lequel le système d'alarme (6) est adapté à émettre une alarme avant que le filtre protecteur (3) soit actionné.

14. Dispositif selon la revendication 13, dans lequel le système d'alarme (6) est adapté à indiquer un paramètre quelconque parmi une direction ou une intensité du rayonnement incident (45).

15. Dispositif selon l'une quelconque des revendications 1 à 14, comprenant en outre une unité de communication (70) qui est adaptée à envoyer et/recevoir des informations concernant le rayonnement laser incident (45).
